# EUROPEAN PATENT APPLICATION

(11) **EP 1 454 620 A2**
(43) Date of publication of application: **08.09.2004**
(21) Application number: 04005448.8
(22) Date of filing: 08.03.2004
(51) Int. Cl.: A61K 7/48

(54) **Skin aging-preventing or improving agent**

(30) Priority: 06.03.2003 JP 2003059467; 25.12.2003 JP 2003431083
(71) Applicant: Kao Corporation, Tokyo 103-8210 (JP)
(72) Inventor: Fujimura, Tsutomu, Tochigi 321-3497 (JP)
(74) Representative: VOSSIUS & PARTNER

(57) **Abstract**

The present invention provides a skin aging-preventing or improving agent. The agent contains a substance capable of enhancing the expression level of Rho kinase or myosin light-chain kinase. According to the present invention, aging of the skin such as sagging of the skin, wrinkle formation, or loss of skin elasticity can be prevented or the skin can be improved.

## Description

### Field of the Invention

The present invention relates to a skin aging-preventing or improving agent; and to a method for preventing aging of the skin or improving the skin.

### Background of the Invention

Aging of cells, particularly aging of cells of the skin, causes changes in appearance, including formation of wrinkles, sagging of the skin, and loss of skin elasticity, and therefore, strong desire exists for preventing aging of the skin or improving the skin. Conventionally, aging of the skin (e.g., formation of wrinkles) has been considered to be strongly related to UV rays, and various studies have been performed on aging of the skin caused by exposure to UV rays (such skin aging is called "photo-aging"). However, cosmetic compositions superseding UV absorbing agents or UV protecting agents have not yet been developed. Meanwhile, collagen-containing cosmetic compositions―which have been developed for prevention of wrinkle formation―have not exhibited sufficient effects.

In recent years, studies have revealed that cytoskeletal proteins such as actin and myosin play a role in generating force in various non-muscle cells (e.g., fibroblasts, vascular endothelial cells, and hepatocytes) as well as in muscle cells, and in controlling cell morphology, etc. Specifically, it has been elucidated that, when generating force, non-muscle cells combine actin filaments with myosin filaments to form fiber structures which are called "stress fibers," and the stress fibers function as myofibrils of non-muscle cells, whereby the non-muscle cells generate force in response to a certain form of stimulation. As has also been reported recently, fibroblasts or vascular endothelial cells generate force by a mechanism similar to that by which muscle cells such as smooth muscle cells generate force, and fibroblasts generate relatively strong force; specifically, a force which is 1/10 to 1/100 that generated by muscle cells, which are intrinsically force-generating cells (see, for example, Kolodney MS, Wysolmerski RB, J. Cell Biol., 117, 73-82 (1992); and Kolodney MS, Elson EL, J. Biol. Chem., 268, 23850-5, 5 (1993)). In individual cells, force generated by the cytoskeletal system, more specifically by stress fibers, is considered to play an important role in, for example, cell division, cell migration, morphological change, and cell adhesion. Also, such force is considered to play an important role *in vivo*; for example, in control of blood flow by vasoconstriction in vascular endothelial cells, and in wound contraction in skin fibroblasts. That is, like the force generated by muscle cells, force generated by non-muscle cells is considered to be actually used in living organisms and function.

Meanwhile, aging has been known to cause muscle weakness, and numerous studies have reported that aging causes reduction of force generated by skeletal muscle cells or a single muscle fiber, or reduction of muscle contraction rate (see, for example, Larsson L, Li X, Frontera W R, Am. J. Physiol., 272, C638-C649 (1997); and Ladora V, Brown M, J. Appl. Physiol., 86, 881-886 (1999)). Therefore, conceivably, force generated by non-muscle cells and its function also closely relate to aging.

However, the relation between force generated by non-muscle cells and aging has not yet been elucidated.

### Summary of the Invention

The present invention relates to a skin aging-preventing or skin-improving agent, which can increase force generated by skin fibroblasts and to a method for preventing aging of the skin or improving the skin. These inventions are based on the finding of the relation between the force generated in skin fibroblasts, i.e. non-muscle cells, and aging.

The present inventors have performed studies on change in force generated by skin fibroblasts with aging, and as a result have found that force generated by skin fibroblasts (i.e., non-muscle cells) is reduced with aging, and that expression of protein of an enzyme which phosphorylates myosin light-chain, i.e. Rho kinase (Rock I, p160 Rock) or myosin light-chain kinase, is reduced in aged skin fibroblasts. The present inventors have also found that a substance capable of enhancing the expression level of such an enzyme can prevent aging of the skin such as sagging of the skin, loss of skin elasticity, or wrinkle formation, or improve the skin.

According to a first aspect of the present invention, there is provided a skin aging-preventing or skin-improving agent which comprises a substance capable of enhancing the expression level of Rho kinase or myosin light-chain kinase.

According to a second aspect of the present invention, there is provided an expression level enhancer for Rho kinase or myosin light-chain kinase, which comprises a plant selected from among *Althaeaofficinalis, Curcuma longa, Actinidia chinensis, Gentiana lutea, Crataeguscuneata, Rehmannia glutinosa, Syzygium aromaticum, Calendula officinalis, Rose canina, Petroselinium sativum, Hamamelisvirginiana, Asiasarum sieboldii, Thymus serpyllum, Hypericum perforatum, Sophora flavescens, Cnidium offcinale, Zizyphus jujuba, Citrus unshiu, Angelica acutiloba, Fucusvesiculosus, Tilia platyllos, Humulus lupulus, Citruslimon, Cassia obatusifolia, Magnolia obovata, Evodia rutaecarpa, Schisandrachinensis, Cornus officinalis, Atractylodes japonica, Digeneasimplex,* and mixtures thereof; or an extract thereof.

According to a third aspect of the present invention, there is provided use of the substance as defined in claim 1 or 2 for the manufacture of a skin aging-preventing or improving agent.

According to a fourth aspect of the present invention, there is provided a method for preventing aging of the skin or improving the skin, which method comprises enhancing the expression level of Rho kinase or myosin light-chain kinase.

According to a fifth aspect of the present invention, there is provided a method for screening skin sagging-improving agents, which method comprises incubating skin fibroblasts with a test substance, and measuring the amount of Rho kinase or myosin light-chain kinase expressed in the fibroblasts.

The agent for preventing aging of the skin or improving the skin of the present invention (hereinafter may be referred to simply as "the present preventing/improving agent") or the agent for enhancing the expression level of Rho kinase or myosin light-chain kinase of the present invention (hereinafter may be referred to simply as "the present expression enhancing agent") exhibits the effect of enhancing the expression level of Rho kinase or myosin light-chain kinase in the skin, whereby force generated by skin fibroblasts is increased, and aging of the skin is prevented or the skin is improved. Therefore, employment of the present preventing/improving agent or the present expression enhancing agent enables prevention of sagging of the skin, wrinkle formation, or loss of skin elasticity, and improvement of the skin. Meanwhile, the method for screening skin sagging-improving agents of the present invention (hereinafter may be referred to simply as "the present screening method") enables simple and efficient screening of substances effective for improving skin sagging.

### Brief Description of the Drawings

Fig. 1 shows reduction of force generated by in-vitro-aged fibroblasts, the force being generated through stimulation by thrombin or LPA; and
Fig. 2 shows change in the amount of myosin light-chain kinase or Rho kinase expressed in fibroblasts in accordance with *in-vitro-*aging of the fibroblasts.

### Detailed Description of the Invention

Unlike the case of muscle cells, non-muscle cells such as skin fibroblasts generally do not contain actin-myosin-polymerized myofibrils which generate force. Therefore, force generation in non-muscle cells requires formation of stress fibers from actin filaments and myosin filaments. In general, actin molecules and myosin molecules are randomly distributed in non-muscle cells, and force is generated through the following process: polymerization of proteins such as actin and myosin induced by phosphorylation of myosin light-chain molecules; formation of stress fibers; energy transmission; and sliding of myosin filaments. Such force generation is considered to be triggered by phosphorylation of myosin light chains, and this phosphorylation is essential for force generation in non-muscle cells (see Kolodney MS, Wysolmerski RB, J. Cell Biol., 117, 73-82 (1992); and Kolodney MS, Elson EL, J. Biol. Chem., 268, 23850-5, 5 (1993)). As has been known, myosin light chains are phosphorylated by means of myosin light-chain kinase or Rho kinase (Rock I, p160 Rock) (see Amano M, Chihara K, Kimura K, Fukata Y, Nakamura N, Matsuura Y, Kaibuchi K., Science, 275, 1308-11 (1997); Totsukawa G, Yamakita Y, Yamashiro S, Hartshorne DJ, Sasaki Y, Matsumura F., J Cell Biol. 150, 797-806 (2000); and *Jikken Igaku* Vol. 17, No. 7 (1999)).

In view of the foregoing, the present inventors focused on such a phosphoenzyme, and performed studies on the relation between the enzyme and the mechanism by which force generation is reduced with aging. The results of the studies revealed that, as described later in Examples 1 and 2, force generated by aged fibroblasts is significantly reduced as compared with the case of young fibroblasts, and, in aged fibroblasts, expression of Rho kinase (Rock I, p160 Rock) and myosin light-chain kinase, which phosphorylate myosin light chains, is reduced as compared with the case of young fibroblasts. Therefore, when expression of Rho kinase or myosin light-chain kinase is increased in fibroblasts, aging of the skin can be prevented or the skin can be improved. A substance capable of enhancing the expression level of Rho kinase or myosin light-chain kinase can serve as a skin aging preventing or improving agent. Meanwhile, when skin fibroblasts are incubated with a test substance, and the amount of Rho kinase or myosin light-chain kinase expressed in the fibroblasts is measured, screening of, for example, skin sagging-improving agents can be performed.

As used herein, the term "Rho kinase" or "myosin light-chain kinase" refers to an enzyme for phosphorylating myosin light chains in non-muscle cells such as fibroblasts, vascular endothelial cells, and hepatocytes, particularly in skin fibroblasts; and the term "substance capable of enhancing the expression level of Rho kinase or myosin light-chain kinase" refers to a substance which can enhance expression of Rho kinase or myosin light-chain kinase and increase force generated by non-muscle cells, particularly by skin fibroblasts, whereby aging of the skin tissue such as sagging of the skin, loss of skin elasticity, or wrinkle formation can be prevented or the skin can be improved.

The substance capable of enhancing the expression level of Rho kinase or myosin light-chain kinase may be a naturally occurring substance or a synthetic compound. Preferred examples of the substance include plants such as *Althaeaofficinalis, Curcuma longa, Actinidia chinensis, Gentiana lutea, Crataeguscuneata, Rehmannia glutinosa, Syzygium aromaticum, Calendula officinalis, Rose canina, Petroselinium sativum, Hamamelisvirginiana, Asiasarum sieboldii, Thymus serpyllum, Hypericum perforatum, Sophora flavescens, Cnidium offcinale, Zizyphus jujuba, Citrus unshiu, Angelica acutiloba, Fucusvesiculosus, Tilia platyllos, Humulus lupulus, Citruslimon, Cassia obatusifolia, Magnolia obovata, Evodia rutaecarpa, Schisandrachinensis, Cornus officinalis, Atractylodes japonica, and Digeneasimplex; and* extracts of these plants.

Of these, the substance capable of enhancing the expression level of Rho kinase is preferably *Althaeaofficinalis, Curcuma longa, Actinidia chinensis, Gentiana lutea, Crataeguscuneata, Rehmannia glutinosa, Syzygium aromaticum, Calendula officinalis, Rose canina, Petroselinium sativum, Hamamelisvirginiana, Hypericum perforatum, Sophora flavescens, Cnidium offcinale, Zizyphus jujuba, Citrus unshiu, Angelica acutiloba, Fucusvesiculosus, Tilia platyllos, Humulus lupulus, Citruslimon, Cassia obatusifolia, Magnolia obovata, Evodia rutaecarpa, Schisandrachinensis, Cornus officinalis, Atractylodes japonica,* and *Digeneasimplex;* and the substance capable of enhancing the expression level of myosin light-chain kinase is preferably *Althaeaofficinalis, Asiasarum sieboldii, Actinidia chinensis, Gentiana lutea, Thymus serpyllum, Syzygium aromaticum, Calendula officinalis, Rose* canina, and *Petroselinium sativum.*

The whole or a part (e.g., leaf, bark, branch, fruit, or root) of any of the aforementioned plants may be employed without any treatment, or may be ground before use. Preferred parts to be employed of the respective plants are as follows: root, rhizome, or leaf for *Althaeaofficinalis;* rhizome for *Curcuma longa;* fruit for *Actinidia chinensis;* root or rhizome for *Gentiana lutea;* fruit for *Crataeguscuneata;* root for *Rehmannia glutinosa;* bud for *Syzygium aromaticum;* head flower for *Calendula officinalis;* fruit for Rose canina; leaf for Petroselinium sativum; leaf or bark for *Hamamelisvirginiana;* root or rhizome for *Asiasarum sieboldii;* aerial part for *Thymus serpyllum;* aerial part for *Hypericum perforatum;* root for *Sophora flavescens;* rhizome for *Cnidium offcinale;* fruit for *Zizyphus jujuba;* fruit skin for *Citrus unshiu;* root for *Angelica acutiloba;* whole plant for *Fucusvesiculosus;* flower or leaf for *Tilia platyllos;* female inflorescence for *Humulus lupulus;* fruit for *Citruslimon;* and the parts described in Japanese Pharmacopoeia for *Cassia obatusifolia, Magnolia obovata, Evodia rutaecarpa, Schisandrachinensis, Cornus officinalis, Atractylodes japonica,* and *Digeneasimplex.*

The extract of such a plant includes a solvent extract prepared by subjecting the plant to extraction treatment at an ambient temperature or elevated temperature, or by use of an extraction apparatus such as a Soxhlet extractor, and also includes a diluted solution, concentrate or dried powder of the solvent extract. The solvent employed for preparing the extract may be a polar solvent or a non-polar solvent. Examples of the solvent include water; alcohols such as methanol, ethanol, propanol, and butanol; polyhydric alcohols such as propylene glycol and butylene glycol; ketones such as acetone and methyl ethyl ketone; esters such as methyl acetate and ethyl acetate; chain-type ethers and cyclic ethers such as tetrahydrofuran and diethyl ether; polyethers such as polyethylene glycol; halogenated hydrocarbons such as dichloromethane, chloroform, and carbon tetrachloride; hydrocarbons such as hexane, cyclohexane, and petroleum ether; aromatic hydrocarbons such as benzene and toluene; and pyridines. A mixture of such solvents may be employed.

The aforementioned extract may be employed without any treatment. Alternatively, the extract may be subjected to dilution, concentration, or freeze-drying, and then prepared into a powder or a paste before use.

The aforementioned extract may be subjected to liquid-liquid partition or a similar technique, to thereby remove inert impurities therefrom. In the present invention, the thus-treated extract is preferably employed. If desired, the resultant extract may be subjected to, for example, deodorization or decoloration by means of a known technique before use.

In the present invention, the aforementioned plants or extracts thereof may be employed in combination of two or more species.

When any of the aforementioned plants is incorporated as an active ingredient into the present preventing/improving agent or the present expression enhancing agent, the amount of the plant as reduced to dry weight is preferably 0.00001 to 5 wt.%, more preferably 0.0001 to 3 wt.% of the agent. Meanwhile, when an extract of the plant is incorporated as an active ingredient, the iamount of the extract as reduced to solid content is preferably 0.00001 to 5 wt.%, more preferably 0.0001 to 2 wt.% of the agent.

The present preventing/improving agent or the present expression enhancing agent may be processed into a variety of preparations; for example, external agents, oral medicines, and injections. Preferably, the present preventing/improving agent or the present expression enhancing agent is used as a drug, a quasi-drug, or an external agent such as a cosmetic composition.

The present preventing/improving agent or the present expression enhancing agent may contain, in addition to the aforementioned plant or an extract thereof, an ingredient such as a UV absorbing agent, a UV protecting agent, collagen, a humectant, an anti-inflammatory agent, or an antioxidant. Preferably, the present preventing/improving agent or the present expression enhancing agent contains a UV absorbing agent and/or a UV protecting agent, from the viewpoint of prevention of aging of the skin or improvement of the skin.

Examples of the UV absorbing agent include a benzophenone UV absorbing agent, a p-aminobenzoic acid UV absorbing agent, a p-methoxycinnamate (e.g., 2-ethylhexyl p-methoxycinnamate) UV absorbing agent, and a salicylic acid UV absorbing agent. Examples of the UV protecting agent include titanium oxide and zinc oxide. The amount of the UV absorbing agent or UV protecting agent incorporated into the present preventing/improving agent is preferably 0.01 to 20 wt.%, more preferably 0.1 to 10 wt.% of the agent, from the viewpoint of prevention of aging of the skin or improvement of the skin.

Examples of the specific product form of the present preventing/improving agent or the present expression enhancing agent include a cream, an ointment, a gel, a lotion, a solution, a pack, and a foundation. When prepared into such a product form, the present preventing/improving agent or the present expression enhancing agent may contain, for example, an oil agent, a surfactant, a gelling agent, a preservative, an antioxidant, a solvent, an alcohol, a chelating agent, a thickener, a colorant, a perfume, or water.

The amount of the present preventing/improving agent or the present expression enhancing agent to be used in practice varies in accordance with the amount of the active ingredient contained in the agent. For example, when the present preventing/improving agent or the present expression enhancing agent is in the form of cream or ointment, preferably, the agent is applied to the skin in an amount of 0.01 to 10 mg per cm² of skin surface. When the present preventing/improving agent or the present expression enhancing agent is in the form of solution, preferably, the agent is applied to the skin in an amount of 0.01 to 10 mg per cm² of skin surface.

The present screening method includes incubating skin fibroblasts with a test substance, and measuring the amount of Rho kinase or myosin light-chain kinase expressed in the fibroblasts.

Culturing of skin fibroblasts is performed under the below-described conditions. Culturing of skin fibroblasts is performed by use of a customary medium in which the fibroblasts can be cultured. Examples of such a medium include Eagle's basal medium (BME), Eagle's minimum essential medium (MEM), Dulbecco's modified Eagle's medium (DMEM), MCDB medium, 199 Earle's medium, and RPMI 1640 modified medium. Particularly, MEM and DMEM are preferred. Fetal bovine serum (FBS), fetal calf serum (FCS), new born calf serum (NBS), or a similar serum is added to such a medium. Particularly preferably, FBS or FCS is added thereto. The amount of such a serum to be added is 3 to 15%, preferably 5 to 10%, during the course of passage or proliferation of skin fibroblasts, whereas the amount of the serum to be added is 0% to 5%, preferably 0.5% to 5%, during the course of incubation of a test substance.

The initial concentration of the skin fibroblasts to be inoculated is preferably 10,000 to 50,000 cells per cm² of dish area. For example, when a dish having a diameter of 60 mm is employed, the concentration of the skin fibroblasts to be inoculated is preferably 270,000 to 1,450,000 cells per dish. The dish to be employed may be a customary culture dish or a collagen-coated dish.

Incubation of skin fibroblasts with a test substance is performed as follows. A test substance in an amount of 0.000001% (evaporation residue wt.%) to 0.1% (evaporation residue wt.%), preferably 0.0001% (evaporation residue wt.%) to 0.01% (evaporation residue wt.%) is added to skin fibroblasts in a medium. In the case where the evaporation residue wt.% of a test substance is unknown, an extract of the test substance is added to skin fibroblasts such that the concentration of the extract is adjusted to 0.0001 vol.% to 5 vol.%, preferably 0.01 vol.% to 5 vol.%. Incubation of the resultant mixture is performed under generally employed conditions for cell culture; for example, incubation is performed in the presence of 5% CO₂ at 37°C for about 24 to about 96 hours, preferably about 24 to about 48 hours.

The amount of expressed Rho kinase or myosin light-chain kinase can be measured by use of an anti-Rho kinase antibody or an anti-myosin light-chain kinase antibody by means of, for example, dot blotting, western blotting, enzyme-linked immunosorbent assay (ELISA), or radioimmunoassay (RIA). Western blotting is more preferred from the viewpoint of convenience.

Preferably, the amount of Rho kinase or myosin light-chain kinase expressed in the above-incubated group is determined by comparing the signal intensity of the incubated group with that of a control group prepared through addition to skin fibroblasts of a solvent containing no test substance, wherein the signal intensity of the control group is taken as 100%. The expression amount can be visually determined by comparing the incubated group with the control group in terms of increase or decrease in signal intensity, but preferably, quantified by means of image analysis processing.

### Examples

### Example 1 Reduction of tension generated by in-vitro-aged fibroblasts

### (1) Cells

Human skin fibroblasts (derived from male abdomen, passage number: 2 to 3) were purchased from Dainippon Pharmaceutical Co., Ltd., and the passage number of the fibroblasts at the time of purchase was taken as 3. After the fibroblasts reached confluency in a DMEM containing 5% FCS, the fibroblasts were subcultured at a split ratio of 1 : 4. Young fibroblasts (passage number: 5 to 7) were compared with old fibroblasts; i.e., *in*-*vitro*-aged fibroblasts (passage number: 16 to 19).

### (2) Measurement of force generated by cells

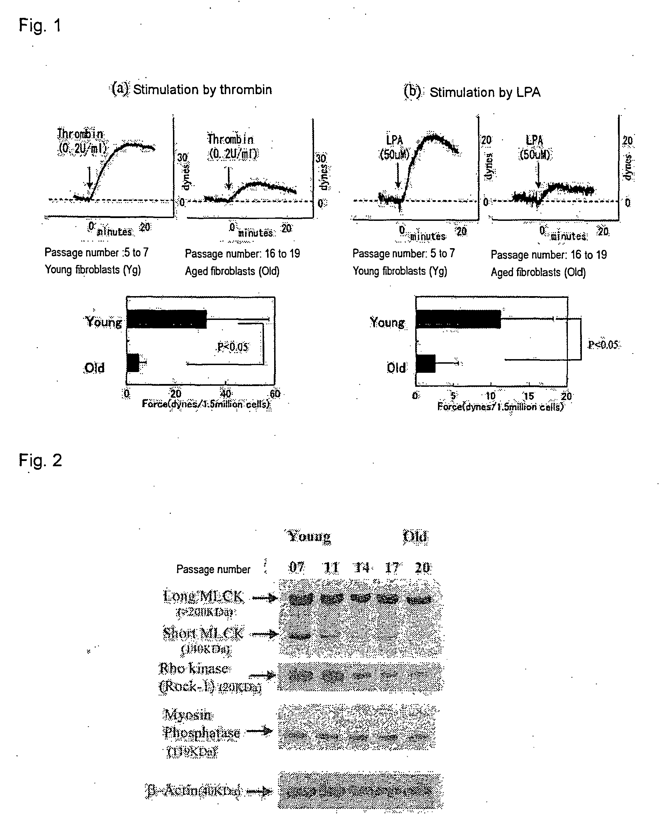

Measurement of force was performed in a collagen gel culture system by means of the method of Kolodeny, et *al*. Specifically, a fibroblasts-embedded collagen gel (1.5 × 10⁶ cells, 1.5 mg/mL collagen, Nitta Gelatin Type I-A) was immobilized and stabilized in a serum-free medium (medium temperature: 37°C) (50 mL), and subsequently 1 mL of a substance (thrombin or lysophosphatidic acid (LPA)) whose concentration had been adjusted by use of a serum-free DMEM to about 50 times its final concentration (final concentration: thrombin = 0.2 U/mL, LPA = 10 µM) was added to the fibroblasts-containing medium. Force generated by the fibroblasts was measured by use of an isotonic transducer and recorded by use of MP 100A-CE (BIOPAC Systems, Santa Barbara). The results are shown in Fig. 1.

As shown in Fig. 1, it was confirmed that the force generated by the *in-vitro*-aged fibroblasts (old fibroblasts), through stimulation by thrombin or LPA was significantly reduced as compared with the force generated by the young fibroblasts. Since the number of the old fibroblasts is equal to that of the young fibroblasts, it is conceived that force generated by individual fibroblasts is reduced with aging of the cells.

### Example 2 Expression of myosin phosphoenzyme in in-vitro-aged fibroblasts

### (1) Cells and antibodies

Human skin fibroblasts employed in Example 1 were in-vitro aged in a manner similar to that of Example 1.

The following antibodies were employed: anti-myosin light-chain kinase antibody (clone; K36, Sigma, M7905) for myosin light-chain kinase; anti-Rock (Rho kinase)-1 (clone; H85, Santa Cruz Biotechnology, Inc.) for Rho kinase; anti-actin antibody (I-19, Santa Cruz Biotechnology, Inc.) for actin; and anti-myosin phosphatase antibody (PRB-457C, Berkeley Antibody Co.) for myosin phosphatase. Peroxidase-labeled Anti-goat IgG and peroxidase-labeled Anti-Rabbit IgG (products of Santa Cruz Biotechnology, Inc.) were employed as secondary antibodies in compliance with the corresponding primary antibodies.

### (2) Western blotting

Human skin fibroblasts were cultured in a collagen Type I-coated dish (IWAKI) (passage number: 7, 11, 13, 17, and 20) until the fibroblasts reached confluency, and each type of the fibroblasts was employed as a sample. The sample was washed with cold PBS and then dissolved in an ice-cooled RIPA buffer (1% NP40, 0.5% sodium cholate, 0.1% SDS/PBS solution), and subsequently, decomposed by sticking a 27G syringe into the sample solution several times and extracted, followed by protein quantification by use of BCA Protein Assay Kit (PIERCE). After the protein content was adjusted, Laemmli buffer (Bio Rad) containing 10% mercaptoethanol was added to the resultant sample, followed by boiling for five minutes.

The resultant sample was applied to wells containing a 7.5%, 12.5%, or 15% polyacrylamide SDS gel (Bio Rad), and then electrophoresed at 60 V for three hours. Thereafter, the electrophoresed sample was blotted onto a nitrocellulose membrane (Bio Rad) for 1.0 to 1.5 hours under ice-cooling with application of a current of 220 to 250 mA. Subsequently, the resultant membrane was subjected to blocking in a 2% skim milk/PBS solution at room temperature for one hour or at 4°C for about 12 hours, and then reacted with the anti-myosin light-chain kinase antibody (2,000-fold diluted), the anti-Rock-1 (anti-Rho kinase, H85, 100-fold diluted), the anti-actin antibody (5,000-fold diluted), and the anti-myosin phosphatase antibody (500-fold diluted) at room temperature for one hour. The resultant membrane was washed with PBS-T (0.1% Tween 20/PBS solution), and then reacted with the peroxidase-labeled secondary antibodies (1,000-fold diluted). Thereafter, the resultant membrane was washed with PBS-T, and then colored by use of ECL kit (Amersham), followed by detection by use of X-ray film (Hyperfilm, Amersham). The results are shown in Fig. 2. The amounts of expressed proteins shown in Fig. 2 were quantified by use of an image analysis processor (Atto Corporation, Densitograph: Lane & Spot Analyzer). The amount of each of the proteins expressed in the youngest cells (passage number: 7) was taken as 100%, and the relative amounts of the proteins expressed in the older cells (passage number: 11, 13, 17, or 20) were calculated on the basis of the values of the youngest cells (Table 1). In Fig. 2 and Table 1, "MLCK" denotes myosin light-chain kinase.

**Table 1**

| | Long MLCK | Short MLCK | Rho kinase | Myosin phosphatase | β-Actin |
|---|---|---|---|---|---|
| 7 | 100 | 100 | 100 | 100 | 100 |
| 11 | 94 | 61 | 86 | 84 | 86 |
| 13 | 82 | 28 | 30 | 80 | 81 |
| 17 | 90 | 45 | 24 | 75 | 81 |
| 20 | 82 | 22 | 18 | 63 | 84 |

As shown in Fig. 2 and Table 1, in the *in-vitro*-aged fibroblasts, expression of myosin light-chain kinase and Rho kinase (Rock I, p160 Rock) is reduced as compared with that in the young fibroblasts. In the case of myosin light-chain kinase which has been known to have two types; i.e., a long type having a molecular weight of 200 kDa or more and a short type having a molecular weight of 140 kDa (Poperechnaya A, Varlamova O, Lin PJ, Stull JT, Bresnick AR., J Cell Biol, 151, 697-707 (2000)), expression of both the types in the in-*vitro-*aged fibroblasts was found to be reduced. In the case of myosin phosphatase (enzyme that dephosphorylates myosin light chains), reduction of expression in the *in-vitro*-aged fibroblasts was found to be less significant as compared with the case of Rho kinase or myosin light-chain kinase. In contrast, in the case of β-actin whose expression amount was quantified for comparison, no significant reduction of expression in the *in-vitro*-aged fibroblasts was observed.

Phosphorylation of myosin light chains promotes myosin polymerization, induces polymerization between actin filaments and myosin filaments to form stress fibers, and activates myosin ATPase, thereby promoting generation of force by cells. It is suggested that reduction of the aforementioned phosphoenzymes leads to reduction of the amount of phosphorylated myosin in the cells, and therefore, force generated by the cells is reduced. The above results reveal that myosin light-chain kinase and Rho kinase are key enzymes in the reduction of force generated by aged fibroblasts.

### Example 3 Agent for enhancing the expression level of myosin light-chain kinase

Aged skin fibroblasts employed in Examples 1 and 2 (passage number: 16 to 19) were inoculated into a 6-well dish, and 24 hours after the inoculation, an extract of a crude drug was added to the fibroblasts such that the extract concentration as reduced to evaporation solid residue was adjusted to 0.0005% to 0.01% (in the case of a crude drug described in Japanese Pharmacopoeia, an extract of the crude drug was added to the fibroblasts such that the extract concentration was adjusted to 0.05 vol.% to 0.2 vol.%), followed by culturing for 48 hours. Thereafter, a sample was prepared in a manner similar to that described in Example 2, and then subjected to western blotting. Subsequently, the amount of myosin light-chain kinase expressed in the above-cultured group was compared with the amount of myosin light-chain kinase expressed in a control group in which the skin fibroblasts were not treated with an extract of a crude drug. The resultant signals were quantified by means of image analysis processing. The results are shown in Table 2.

**Table 2**

| | Concentration % (as reduced to evaporation solid residue) | Myosin light-chain kinase | |
|---|---|---|---|
| | | Long type | Short type |
| *Asiasarum sieboldii* | 0.002 | 114 | 100 |
| *Althaea officinalis* | 0.002 | 100 | 167 |
| *Actinidia chinensis* | 0.002 | 149 | 179 |
| *Gentiana lutea* | 0.002 | 139 | 178 |
| *Thymus serpyllum* | 0.002 | 142 | 100 |
| *Syzygium aromaticum* | 0.002 | 155 | 205 |
| *Calendula officinalis* | 0.002 | 159 | 211 |
| *Rose canina* | 0.002 | 183 | 208 |
| *Petroselinium sativum* | 0.002 | 155 | 219 |
| Control | | 100 | 100 |

As is clear from Table 2, each of the above-employed crude drugs increases the amount of myosin light-chain kinase expressed in the skin fibroblasts.

### Example 4 Agent for enhancing the expression level of Rho kinase

In a manner similar to that of Example 3, the amount of Rho kinase expressed in a group in which the skin fibroblasts were cultured with an extract of a crude drug was compared with the amount of Rho kinase expressed in a control group in which the skin fibroblasts were not treated with an extract of a crude drug. As is clear from Table 3, each of the employed crude drugs increases the amount of Rho kinase expressed in the skin fibroblasts.

**Table 3**

| | Concentration | Rho kinase |
|---|---|---|
| *Althaeaofficinalis* | 0.002 (evaporation solid residue %) | 175 |
| *Curcuma longa* | 0.002 (evaporation solid residue %) | 207 |
| *Actinidia chinensis* | 0.002 (evaporation solid residue %) | 196 |
| *Gentiana lutea* | 0.002 (evaporation solid residue %) | 231 |
| *Crataeguscuneata* | 0.002 (evaporation solid residue %) | 284 |
| *Rehmannia glutinosa* | 0.002 (evaporation solid residue %) | 356 |
| *Syzygium aromaticum* | 0.002 (evaporation solid residue %) | 162 |
| *Calendula officinalis* | 0.002 (evaporation solid residue %) | 202 |
| *Rose canina* | 0.002 (evaporation solid residue %) | 264 |
| *Petroselinium sativum* | 0.002 (evaporation solid residue %) | 236 |
| *Hamamelisvirginiana* | 0.002 (evaporation solid residue %) | 232 |
| *Hypericum perforatum* | 0.002 (evaporation solid residue %) | 139 |
| *Sophora flavescens* | 0.002 (evaporation solid residue %) | 218 |
| | 0.01 (evaporation solid residue %) | 183 |
| *Cnidium offcinale* | 0.002 (evaporation solid residue %) | 134 |
| | 0.01 (evaporation solid residue %) | 135 |
| *Zizyphus jujuba* | 0.0005 (evaporation solid residue %) | 125 |
| | 0.002 (evaporation solid residue %) | 135 |
| *Citrus unshiu* | 0.002 (evaporation solid residue %) | 123 |
| | 0.01 (evaporation solid residue %) | 116 |
| *Angelica acutiloba* | 0.002 (evaporation solid residue %) | 323 |
| | 0.01 (evaporation solid residue %) | 143 |
| *Fucusvesiculosus* | 0.002 (evaporation solid residue %) | 145 |
| *Tilia platyllos* | 0.0005 (evaporation solid residue %) | 120 |
| | 0.002 (evaporation solid residue %) | 114 |
| *Citruslimon* | 0.002 (evaporation solid residue %) | 144 |
| | 0.01 (evaporation solid residue %) | 141 |
| *Cassia obatusifolia* | 0.05 (vol.%) | 135 |
| | 0.2 (vol.%) | 113 |
| *Magnolia obovata* | 0.002 (vol.%) | 127 |
| | 0.01 (vol.%) | 136 |
| *Evodia rutaecarpa* | 0.002 (vol.%) | 165 |
| | 0.01 (vol.%) | 109 |
| *Schisandrachinensis* | 0.05 (vol.%) | 119 |
| | 0.2 (vol.%) | 128 |
| *Cornus officinalis* | 0.05 (vol.%) | 141 |
| | 0.2 (vol.%) | 159 |
| *Atractylodes japonica* | 0.05 (vol.%) | 115 |
| | 0.2 (vol.%) | 113 |
| *Digeneasimplex* | 0.2 (vol.%) | 123 |
| Control | | 100 |

Table 4 shows the plant extracts employed in Examples 3 and 5. The extracts were prepared by means of a customary method.

**Table 4**

| | Part employed | Extraction solvent |
|---|---|---|
| *Althaeaofficinalis* | Root/Leaf | 50% Ethanol |
| *Curcuma longa* | Rhizome | 50% Ethanol |
| *Actinidia chinensis* | Fruit | Water |
| *Gentiana lutea* | Root | 50% Ethanol |
| *Crataeguscuneata* | Fruit | 50% Ethanol |
| *Rehmannia glutinosa* | Root | 50% Ethanol |
| *Syzygium aromaticum* | Bud | 100% Ethanol |
| *Calendula officinalis* | Head inflorescence | 50% Ethanol |
| *Rose canina* | Fruit | 50% Ethanol |
| *Petroselinium sativum* | Leaf | 50% 1,3-Butylene glycol (BG) |
| *Hamamelisvirginiana* | Leaf | 50% 1,3-Butylene glycol (BG) |
| *Asiasarum sieboldii* | Root/Rhizome | Water |
| *Thymus serpyllum* | Leaf/Stem | 50% Ethanol |
| *Hypericum perforatum* | Aerial part | 45% 1,3-Butylene glycol |
| *Sophora flavescens* | Root | 50% Ethanol |
| *Cnidium offcinale* | Rhizome | 50% Ethanol |
| *Zizyphus jujuba* | Fruit | 30% Ethanol |
| *Citrus unshiu* | Fruit skin | 50% Ethanol |
| *Angelica acutiloba* | Root | 50% Ethanol |
| *Fucusvesiculosus* | Whole plant | 45% 1,3-Butylene glycol |
| *Tilia platyllos* | Flower/Leaf | 50% 1,3-Butylene glycol |
| *Humulus lupulus* | Female flower bud | 50% Ethanol/ 20% 1,3-Butylene glycol |
| *Citruslimon* | Fruit | 30% Ethanol |
| *Cassia obatusifolia* | # | 50% Ethanol |
| *Magnolia obovata* | # | 50% Ethanol |
| *Evodia rutaecarpa* | # | 50% Ethanol |
| *Schisandrachinensis* | # | 50% Ethanol |
| *Cornus officinalis* | # | 50% Ethanol |
| *Atractylodes japonica* | # | 50% Ethanol |
| *Digeneasimplex* | # | 50% Ethanol |
| #: Crude drug described in Japanese Pharmacopoeia | | |

### EXAMPLE 5

### Effect of plants extract enhancing the expression level of Rho kinase on wrinkle smoothing

Female HR/ICR hairless mice were used in this example. This strain was established from a cross between the hairless strain HR/HR (Skh-1) and the normal-haired strain HaM/ICR, and has been maintained in our laboratory by breeding hairless brothers and phenotypically haired sisters. Mice (8-10 animals per cage) were irradiated using SE20 lamps (Toshiba Co., Ltd., Japan) as the UVB source 5 times a week (daily) for 12 weeks. A progressive UVB exposure regimen was used starting at approximately 47 mJ/cm² per exposure at week 1, which was then increased by 6-7 mJ/cm² per week until week 4. That exposure dose (67 mJ/cm²) was then kept constant for the remaining period of exposure. The amount of energy was measured by a UV-radiometer (UVR-305/365D, Tokyo Optical K.K.). The initial dose of 67 mJ/cm² was just below the erythemal dose.

After confirming that hairless mice had got wrinkles at their backs, they were divided into groups each consisting of 6-8 mice. The number of times of UVB exposure was diminished to 3 times a week. And aqueous ethanol (water/ethanol=20/80 vol. %) solutions containing 3 vol. % of plant extracts shown in Table 5 *(Gentiana lutea, Althaea officinalis, Fucus vesiculasus,* and *Curcuma longa)* were applied to dorsal skin twice a day, 5 days a week for 4 weeks in a dose of 100 micro L. As a vehicle control, aqueous ethanol (water/ethanol=20/80 vol %) was applied in a dose of 100 micro L like the samples.

Before (0 week) and after (4 weeks) application, the degree of wrinkles was visually observed to evaluate the samples in accordance with the following standard (wrinkle scores). Intermediate score (1.5, 2.5, 3.5) was also used in this evaluation.

Wrinkle score: 1: No wrinkles were observed (Wrinkles were completely removed or smoothed); 2: Wrinkles were observed weakly; 3: Wrinkles were observed moderately; 4: Wrinkles were observed severely. Difference of wrinkle scores between control and sample (Effectiveness of Sample) were determined by the calculating scale shown below : (Sample wrinkle score (before) -Sample wrinkle score (after)) -(Control wrinkle score (before) -Control wrinkle score (after))

The results are shown in Table 1.

| Table 5 | | | |
|---|---|---|---|
| | Wrinke score (before) mean±SD | Wrinkle score (after) mean±SD | Difference of wrinkle scores between control and sample (Effectiveness of Sample) |
| Control (vehicle) | 2.7142±0.393 4 | 2.8571±0.475 6 | 0.00 |
| *Gentiana lutea* | 2.75±0.4183 | 2.5833±0.376 4 | 0.31 |
| *Althaea officinali s* | 2.7±0.2738 | 2.6±0.4183 | 0.24 |
| *Fucus vesiculosu s* | 2.7±0.5701 | 1.8±0.2739 | 1.04 |
| *Curcuma longa* | 2.6666±0.516 4 | 2.3±0.5701 | 0.51 |

As is clear from Table 5, each of the plant extracts enhancing the expression level of Rho kinase and/or myosin light chain kinase (*Gentiana lutea* and *Althaea officinalis*). has wrinkle smoothing efficacy.

## Claims

1. A skin aging-preventing or improving agent which comprises a substance capable of enhancing the expression level of Rho kinase or myosin light-chain kinase.

2. The skin aging-preventing or improving agent according to claim 1, wherein the substance capable of enhancing the expression level of Rho kinase or myosin light-chain kinase is a plant selected from the group consisting of *Althaeaofficinalis, Curcuma longa, Actinidia chinensis, Gentiana lutea, Crataeguscuneata, Rehmannia glutinosa, Syzygium aromaticum, Calendula officinalis, Rose canina, Petroselinium sativum, Hamamelisvirginiana, Asiasarum sieboldii, Thymus serpyllum, Hypericum perforatum, Sophora flavescens, Cnidium offcinale, Zizyphus jujuba, Citrus unshiu, Angelica acutiloba, Fucusvesiculosus, Tilia platyllos, Humulus lupulus, Citruslimon, Cassia obatusifolia, Magnolia obovata, Evodia rutaecarpa, Schisandrachinensis, Cornus officinalis, Atractylodes japonica, Digeneasimplex,* and mixtures thereof; or an extract thereof.

3. An expression level enhancer for Rho kinase or myosin light-chain kinase, which comprises a plant selected from the group consisting of *Althaeaofficinalis, Curcuma longa, Actinidia chinensis, Gentiana lutea, Crataeguscuneata, Rehmannia glutinosa, Syzygium aromaticum, Calendula officinalis, Rose canina, Petroselinium sativum, Hamamelisvirginiana, Asiasarum sieboldii, Thymus serpyllum, Hypericum perforatum, Sophora flavescens, Cnidium offcinale,* *Zizyphus jujuba, Citrus unshiu, Angelica acutiloba, Fucusvesiculosus, Tilia platyllos, Humulus lupulus, Citruslimon, Cassia obatusifolia, Magnolia obovata, Evodia rutaecarpa, Schisandrachinensis, Cornus officinalis, Atractylodes japonica, Digeneasimplex, and mixtures thereof;* or an extract thereof.

4. Use of the substance as defined in claim 1 or 2 for the manufacture of a skin aging-preventing or improving agent.

5. A method for preventing aging of the skin or improving the skin, which method comprises enhancing expression level of Rho kinase or myosin light-chain kinase.

6. A method for screening skin wrinkling- and sagging-improving agents, which method comprises incubating skin fibroblasts with a test substance, and measuring the amount of Rho kinase or myosin light-chain kinase expressed in the fibroblasts.
